# EUROPÄISCHE PATENTANMELDUNG

(11) **EP 0 959 174 A1**
(43) Veröffentlichungstag der Anmeldung: **24.11.1999**
(21) Anmeldenummer: 98810458.4
(22) Anmeldetag: 18.05.1998
(51) Int. Cl.: D06P 5/06, D06P 1/642, C07D 273/04, C07D 251/08

(54) **Verfahren zur Behandlung von natürlichen oder synthetischen Polyamidfasermaterialien**

(71) Anmelder: Ciba Specialty Chemicals Holding Inc., 4057 Basel (CH)
(72) Erfinder: Adam, Jean-Marie, 68300 Rosenau (FR)

(57) **Zusammenfassung**

Verfahren zur Verbesserung der Beständigkeit von Farbstoffen auf natürlichen oder synthetischen Polyamidfasermaterialien gegen Ozoneinwirkung, dadurch gekennzeichnet, dass man das Fasermaterial vor, während oder nach der Färbung mit einer Flotte, die mindestens eine Verbindung der Formel (1) 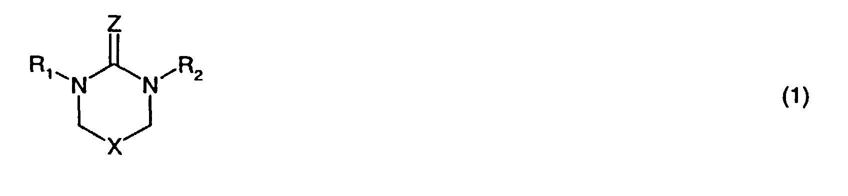 enthält, worin
X Sauerstoff oder einen Rest N-R₃ und
Z Sauerstoff oder Schwefel bedeuten, und
R₁, R₂ und R₃ unabhängig voneinander Wasserstoff, gegebenenfalls substituiertes C₁-C₆-Alkyl, C₅-C₇-CyCloalkyl oder C₇-C₁₂-Phenalkyl sind,
   nach Auszieh- oder Kontinueverfahren behandelt.

Die erhaltenen Färbungen und Drucke zeichnen sich durch verbesserte Ozonechtheiten ohne Beeinflussung der Nuance, Farbausbeute und anderen Echtheiten wie beispielsweise der Lichtechtheit aus.

## Beschreibung

Die vorliegende Erfindung betrifft ein Verfahren zur Behandlung von natürlichen oder synthetischen Polyamidfasermaterialien zur Verbesserung der Beständigkeit von Farbstoffen auf diesen Fasermaterialien gegen Ozoneinwirkung.

Färbungen und Drucke mit Farbstoffen zeigen oftmals eine hohe Empfindlichkeit gegen Ozon. Beispielsweise werden Anthrachinonfarbstoffe leicht durch Ozon oxidativ abgebaut und verändern auf diese Weise ihr Absorbtionsverhalten und somit die Farbe. Dieses Verhalten wird insbesondere bei blauen Anthrachinonfarbstoffen beobachtet. Die Nuance einer Trichromiefärbung auf der Basis blauer Anthrachinonfarbstoffe, beispielsweise eines Polyamidteppichgewebes, wird leicht durch Ozoneinwirkung verändert. Man begegnet diesem Mangel im allgemeinen, indem man das gefärbte Polyamidfasermaterial mit Harzen auf der Basis von Phenol-Formaldehyd-Kondensaten oder mit bestimmten niedermolekularen, organischen Verbindungen behandelt. Die bekannten Mittel zur Verbesserung der Ozonbeständigkeit weisen jedoch Nachteile, z.B. eine mangelnde Wirksamkeit oder eine negative Beeinflussung anderer Echtheiten, z.B. der Lichtechtheit auf oder die Verfahren zur Anwendung dieser Mittel sind aufwendig. Daher besteht einerseits ein Bedarf nach verbesserten Mitteln zur Erhöhung der Ozonbeständigkeit von insbesondere anionischen Farbstoffen auf natürlichen oder synthetischen Polyamidfasermaterialien, welche die genannten Nachteile nicht aufweisen. Andererseits besteht ein Bedarf nach einfachen Verfahren zur Behandlung dieser Fasermaterialien.

Es wurde nun gefunden, dass man die Beständigkeit gegen Ozon von beispielsweise Färbungen anionischer Farbstoffe auf Polyamidfasermaterial ohne negative Beeinflussung anderer Echtheiten verbessern kann, wenn man sie einer Behandlung mit den nachfolgend genannten Verbindungen in der beschriebenen Weise unterzieht.

Gegenstand der vorliegenden Erfindung ist daher ein Verfahren zur Verbesserung der Beständigkeit von Farbstoffen auf natürlichen oder synthetischen Polyamidfasermaterialien gegen Ozoneinwirkung, dadurch gekennzeichnet, dass man das Fasermaterial vor, während oder nach der Färbung mit einer Flotte, die mindestens eine Verbindung der Formel (1) 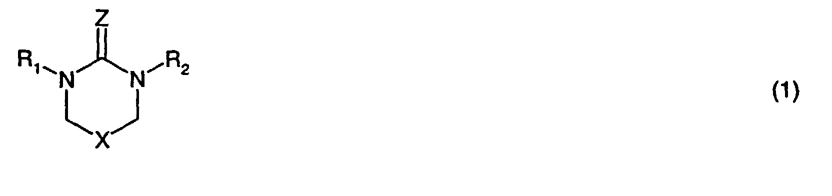 enthält, worin
X Sauerstoff oder einen Rest N-R₃ und
Z Sauerstoff oder Schwefel bedeuten, und
R₁, R₂ und R₃ unabhängig voneinander Wasserstoff, gegebenenfalls substituiertes C₁-C₆-Alkyl, C₅-C₇-Cycloalkyl oder C₇-C₁₂-Phenalkyl sind,
   nach dem Auszieh- oder Kontinueverfahren behandelt.

Als C₁-C₆-Alkyl kommen für R₁, R₂ und R₃ unabhängig voneinander z.B. Methyl, Ethyl, Propyl, Isopropyl, n-Butyl, sek.-Butyl, tert-Butyl, Isobutyl, n-Pentyl, 2-Pentyl, Isopentyl oder n-Hexyl in Betracht. Die genannten Alkylreste können unsubstituiert oder z.B. durch Halogen, wie z.B. Chlor oder Brom; Hydroxy; C₁-C₄-Alkoxy, wie z.B. Methoxy oder Ethoxy; C₂-C₄-Alkanoylamino, wie z.B. Acetylamino oder Propionylamino; Sulfo; oder Sulfato substituiert sein. Bevorzugt als Alkylreste sind die entsprechenden unsubstituierten Reste oder die durch Sulfo substituierten Reste.

Als C₅-C₇-Cycloalkyl kommen für R₁, R₂ und R₃ unabhängig voneinander z.B. Cyclopentyl oder Cyclohexyl in Frage. Die genannten Alkylreste können unsubstituiert oder z.B. durch C₁-C₄-Alkyl, wie z.B. Methyl oder Ethyl, substituiert sein. Bevorzugt sind die unsubstituierten Cycloalkylreste.

Als C₇-C₁₂-Phenalkyl kommen für R₁, R₂ und R₃ unabhängig voneinander z.B. Benzyl, 2-Phenylethyl, 3-Phenylpropyl, Cumyl, oder 4-Phenylbut-1-yl oder -2-yl in Betracht. Bevorzugt ist der Benzyl-, 2-Phenylethyl- oder der 4-Phenylbut-2-ylrest und insbesondere der Benzylrest. Die genannten Phenalkylreste können unsubstituiert oder z.B. im Phenylteil durch Halogen, wie z.B. Chlor oder Brom; C₁-C₄-Alkyl, wie z.B. Methyl oder Ethyl; C₁-C₄-Alkoxy, wie z.B. Methoxy oder Ethoxy; C₂-C₄-Alkanoylamino, wie z.B. Acetylamino oder Propionylamino; oder Sulfo substituiert sein. Bevorzugt als Phenalkylreste sind die entsprechenden unsubstituierten Reste oder die im Phenylteil durch Sulfo substituierten Reste.

Es können auch Gemische von mehreren der zuvor genannten Verbindungen der Formel (1) verwendet werden.

Die erfindungsgemäss als Mittel zur Verbesserung der Ozonbeständigkeit verwendeten Verbindungen sind bekannt oder die Verbindungen können in Analogie zu bekannten Verfahren hergestellt werden.

Verbindungen der Formel (1), worin X Sauerstoff bedeutet lassen sich beispielsweise herstellen, indem man Harnstoff, Thioharnstoff oder deren N-substituierten Derivate mit Formalin (wässrige Formaldehydlösung) in einem organischen Lösungsmittel, wie z.B. Tetrahydrofuran oder Toluol, in Gegenwart einer Säure, wie z.B. Salzsäure oder Toluolsulfonsäure, bei 40 bis 100°C umsetzt.

Verbindungen der Formel (1), worin X einen Rest N-R₃ bedeutet lassen sich beispielsweise herstellen, indem man Harnstoff, Thioharnstoff oder deren N-substituierten Derivate mit Formalin, gegebenenfalls in einem organischen Lösungsmittel, wie z.B. Tetrahydrofuran oder Toluol, in Gegenwart eines Amins der Formel H₂N-R₃, wobei R₃ die oben angegebenen Bedeutungen und Bevorzugungen hat, bei 40 bis 100°C umsetzt.

Zur Einführung der Sulfogruppe in den Phenylring der Phenalkylreste R₁, R₂ und/oder R₃ werden die Verbindungen der Formel (1) beispielsweise in Oleum bei etwa 0°C gelöst und dann bei Raumtemperatur mehrere Stunden gerührt.

Die N-substituierten Harnstoff- oder Thioharnstoffderivate sind bekannt oder können in Analogie zu bekannten Verfahren hergestellt werden.

Die im erfindungsgemässen Verfahren verwendeten Verbindungen der Formel (1) werden unabhängig vom Flottenverhältnis z.B. in einer Menge von 0,05 bis 10 Gew.-%, vorzugsweise 0,1 bis 6 Gew.-% und besonders bevorzugt 0,5 bis 4 Gew.-%, bezogen auf das Gewicht des Polyamidfasermaterials, eingesetzt.

Die Behandlung des Polyamidfasermaterials mit den erfindungsgemäss verwendeten Verbindungen der Formel (1) kann vor, während oder nach dem Färben und vorzugsweise während oder nach dem Färben erfolgen.

Erfolgt die Behandlung des Polyamidfasermaterials mit den erfindungsgemäss verwendeten Verbindungen der Formel (1) während des Färbevorgangs, so führt man das erfindungsgemässe Verfahren vorteilhafterweise so aus, dass man die Verbindungen der Formel (1) der Färbeflotte in der zuvor angegebenen Menge zusetzt und das Fasermaterial in üblicher Weise nach dem Auszieh- oder Kontinueverfahren und vorzugsweise nach dem Ausziehverfahren färbt.

Erfolgt die Behandlung des Polyamidfasermaterials mit den erfindungsgemäss verwendeten Verbindungen der Formel (1) nach dem Färben, so führt man das erfindungsgemässe Verfahren vorteilhafterweise so aus, dass man zunächst das Polyamidfasermaterial in üblicher Weise färbt und anschliessend eine Nachbehandlung mit einer die Verbindungen der Formel (1) in der zuvor angegebenen Menge enthaltenden frischen wässrigen Flotte anschliesst Danach kann das gefärbte Polyamidfasermaterial ohne weiteren Spülvorgang entwässert und auf übliche Weise getrocknet werden. Die Nachbehandlung erfolgt in der Regel in frischer Flotte. Sie ist aber auch direkt im Färbebad durchführbar, sofern das Färbebad am Ende weitgehend ausgezogen und noch genügend sauer ist. Im Anschluss an die Behandlung wird in der Regel mit Wasser kurz kalt gespült.

Als Polyamidfasermaterial kommt natürliches Polyamidfasermaterial, wie z.B. Wolle oder Seide, oder synthetisches Polyamidfasermaterial, wie z.B. Polyamid 6 oder Polyamid 6.6, oder Fasermischungen wie z.B. Wolle/Cellulose- oder Polyamid/Cellulose-Mischfasern oder Polyamid/Wolle-Mischfasern in Betracht. Bevorzugt handelt es sich bei dem Fasermaterial um synthetisches Polyamidfasermaterial.

Das Textilgut ist in jeglicher Form anwendbar, z.B. als Faser, Garn, Gewebe oder Gewirke.

Die Färbungen erfolgen beispielsweise mit anionischen Farbstoffen, wobei alle üblichen anionischen Farbstoffe, wie sie z.B. in Colour Index, 3. Auflage (1971) sowie den Nachträgen dazu unter den Rubriken "Acid Dyes" oder in den Patentschriften US-A-5,725,606, US-A-5,691,459, US-A-5,650,497, US-A-5,630,851, US-A-5,527,889, US-A-5,234,467, US-A-5,131,919, US-A-5,094,665, US-A-5,092,905 und US-A-2,844,597 beschrieben sind, in Frage kommen.

Beispiele sind sulfogruppenhaltige Monoazo-, Polyazo-, Metallkomplexazo-, Anthrachinon-, Phthalocyanin- oder Formazanfarbstoffe.

Bevorzugt erfolgen die Färbungen mit Anthrachinonfarbstoffen und insbesondere mit blauen Anthrachinonfarbstoffen.

Die zum Färben des Polyamidfasermaterials verwendeten anionischen Farbstoffe liegen entweder in der Form ihrer freien Sulfonsäure oder vorzugsweise als deren Salze vor.

Als Salze kommen beispielsweise die Alkali-, Erdalkali- oder Ammoniumsalze oder die Salze eines organischen Amins in Betracht. Als Beispiele seien die Natrium-, Lithium-, Kalium- oder Ammoniumsalze oder das Salz des Mono-, Di- oder Triäthanolamins genannt.

Die zum Färben des Polyamidfasermaterials verwendeten anionischen Farbstoffe können weitere Zusätze, wie z.B. Kochsalz oder Dextrin, enthalten.

Das Färben des Polyamidfasermaterials mit anionischen Farbstoffen kann gemäss den für diese Farbstoffe üblichen Färbe- bzw. Druckverfahren, beispielsweise nach dem Ausziehverfahren, Foulard- oder Kontinueverfahren, durchgeführt werden. Die Färbeflotten oder Druckpasten können ausser Wasser und den Farbstoffen weitere Zusätze, beispielsweise Netzmittel, Antischaummittel, Egalisiermittel oder die Eigenschaft des Textilmaterials beeinflussende Mittel wie z.B. Weichmachungsmittel, Zusätze zum Flammfestausrüsten oder schmutz-, wasser- und öl-abweisende Mittel sowie wasserenthärtende Mittel und natürliche oder synthetische Verdicker, wie z.B. Alginate und Celluloseäther, enthalten.

Die Mengen, in denen anionische Farbstoffe in den Färbebädern oder Druckpasten verwendet werden, können je nach der gewünschten Farbtiefe in weiten Grenzen schwanken, im allgemeinen haben sich Mengen von 0,01 bis 15 Gew.-%, insbesondere 0,01 bis 10 Gew.-%, bezogen auf das Färbegut bzw. die Druckpaste, als vorteilhaft erwiesen.

Vorzugsweise erfolgt das Färben mit anionischen Farbstoffen bei einem pH-Wert von 3 bis 7, insbesondere 4 bis 7, wobei man in Gegenwart der erfindungsgemäss verwendeten Verbindungen der Formel (1) vorzugsweise bei einem pH-Wert von 2 bis 7 und insbesondere 4 bis 7 färbt. Das Flottenverhältnis kann innerhalb eines weiten Bereichs gewählt werden, z.B. von 1:5 bis 1:50, vorzugsweise 1:5 bis 1:30. Vorzugsweise färbt man bei einer Temperatur von 70 bis 110°C, insbesondere 80 bis 105°C, wobei in Gegenwart der erfindungsgemäss verwendeten Verbindungen der Formel (1) vorzugsweise bei 50 bis 100°C und insbesondere bei 80 bis 100°C gefärbt wird.

Die Nachbehandlung mit den erfindungsgemäss verwendeten Verbindungen der Formel (1) erfolgt vorzugsweise nach dem Ausziehverfahren. Das Flottenverhältnis kann dabei innerhalb eines weiten Bereichs gewählt werden und beträgt z.B. 1:4 bis 1:100, vorzugsweise 1:10 bis 1:40 und insbesondere 1:5 bis 1:40.

Besondere Vorrichtungen sind nicht erforderlich. Es können z.B. die üblichen Färbeapparate, z.B. offene Bäder, Haspelkufen, Jigger oder Paddel-, Düsen- oder Zirkulationsapparate, verwendet werden.

Man arbeitet zweckmässig bei einer Temperatur von z.B. 20 bis 100°C, vorzugsweise 50 bis 100°C und insbesondere 60 bis 100°C. Die Behandlungszeit kann z.B. 10 bis 60 Minuten und vorzugsweise 15 bis 40 Minuten betragen. Der pH-Wert der Flotte liegt in der Regel bei 2 bis 7, vorzugsweise bei 4 bis 7 und insbesondere bei 4 bis 6.

Die Flotte kann ausser dem Fixiermittel weitere übliche Zusätze, z.B. Elektrolyte wie z.B. Natriumchlorid oder Natriumsulfat, Dispergier- und Netzmittel sowie Entschäumer, enthalten.

Man erhält nach dem erfindungsgemässen Verfahren Färbungen oder Drucke von Farbstoffen, z.B. anionischen Farbstoffen, auf Polyamidfasermaterial, welche eine erhebliche Verbesserung der Ozonechtheit aufweisen, ohne dass die Farbausbeute, Nuance oder die Lichtechtheiten negativ beeinflusst werden.

Gegenstand der vorliegenden Erfindung sind ferner Verbindungen der Formel (2) 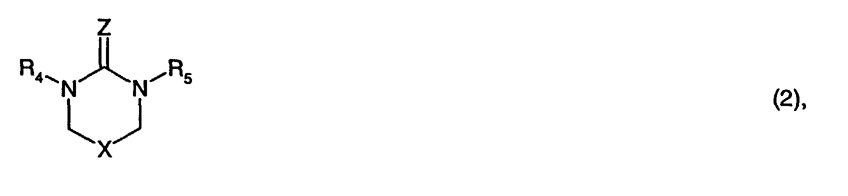 worin
X Sauerstoff oder einen Rest N-R₆ und
Z Sauerstoff oder Schwefel bedeuten, und
R₄, R₅ und R₆ unabhängig voneinander Wasserstoff, gegebenenfalls durch Halogen, Hydroxy, C₁-C₄-Alkoxy, C₂-C₄-Alkanoylamino, Sulfo oder Sulfato substituiertes C₁-C₆-Alkyl, gegebenenfalls durch C₁-C₄-Alkyl substituiertes C₅-C₇-Cycloalkyl oder gegebenenfalls im Phenylteil durch Halogen, C₁-C₄-Alkyl, C₁-C₄-Alkoxy, C₂-C₄-Alkanoylamino oder Sulfo substituiertes C₇-C₁₂-Phenalkyl sind,
   mit der Massgabe, dass
   mindestens einer der Substituenten R₄ und R₅ gegebenenfalls im Phenylteil durch Halogen, C₁-C₄-Alkyl, C₁-C₄-Alkoxy oder C₂-C₄-Alkanoylamino substituiertes C₇-C₁₂-Phenalkyl bedeutet, oder
   mindestens einer der Substituenten R₄, R₅ und R₆ durch Sulfo substituiertes C₁-C₆-Alkyl oder im Phenylteil durch Sulfo substituiertes C₇-C₁₂-Phenalkyl bedeutet.

Bevorzugt sind Verbindungen der Formel (2), worin
R₄, R₅ und R₆ unabhängig voneinander Wasserstoff, gegebenenfalls durch Hydroxy, C₁-C₄-Alkoxy, Sulfo oder Sulfato substituiertes C₁-C₄-Alkyl oder gegebenenfalls im Phenylteil durch C₁-C₄-Alkyl, C₁-C₄-Alkoxy, C₂-C₄-Alkanoylamino oder Sulfo substituiertes C₇-C₁₀-Phenalkyl sind,
   mit der Massgabe, dass
   mindestens einer der Substituenten R₄ und R₅ gegebenenfalls im Phenylteil durch C₁-C₄-Alkyl, C₁-C₄-Alkoxy oder C₂-C₄-Alkanoylamino substituiertes C₇-C₁₀-Phenalkyl bedeutet, oder mindestens einer der Substituenten R₄, R₅ und R₆ durch Sulfo substituiertes C₁-C₄-Alkyl oder im Phenylteil durch Sulfo substituiertes C₇-C₁₀-Phenalkyl bedeutet.

Besonders bevorzugt sind Verbindungen der Formel (2), worin
R₄ gegebenenfalls im Phenylteil durch C₁-C₄-Alkyl, C₁-C₄-Alkoxy oder Sulfo substituiertes C₇-C₁₀-Phenalkyl ist, und
R₅ und R₆ unabhängig voneinander Wasserstoff, gegebenenfalls durch C₁-C₄-Alkoxy oder Sulfo substituiertes C₁-C₄-Alkyl oder gegebenenfalls im Phenylteil durch C₁-C₄-Alkyl, C₁-C₄-Alkoxy oder Sulfo substituiertes C₇-C₁₀-Phenalkyl sind.

Ganz besonders bevorzugt sind Verbindungen der Formel (2), worin
R₄ gegebenenfalls im Phenylteil durch Sulfo substituiertes C₇-C₁₀-Phenalkyl ist, und
R₅ und R₆ unabhängig voneinander Wasserstoff, gegebenenfalls durch Sulfo substituiertes C₁-C₄-Alkyl oder gegebenenfalls im Phenylteil durch Sulfo substituiertes C₇-C₁₀-Phenalkyl sind.

Wichtig sind Verbindungen der Formel (2), worin
Z Schwefel bedeutet.

In einer besonders wichtigen Ausführungsform der vorliegenden Erfindung ist mindestens einer der Substituenten R₄, R₅ und R₆ der Verbindung der Formel (2) als Alkylrest oder Phenalkylrest durch Sulfo substituiert.

R₄, R₅ und R₆ haben weiterhin die Bedeutungen und Bevorzugungen, die oben im Zusammenhang mit den verfahrensgemäss verwendeten Verbindungen der Formel (1) für die Reste R₁, R₂ und R₃ angegeben sind.

Einen weiteren Gegenstand dervorliegenden Erfindung bildet ein Verfahren zur Herstellung der erfindungsgemässen Verbindungen der Formel (2), dadurch gekennzeichnet, dass man eine Verbindung der Formel (3) 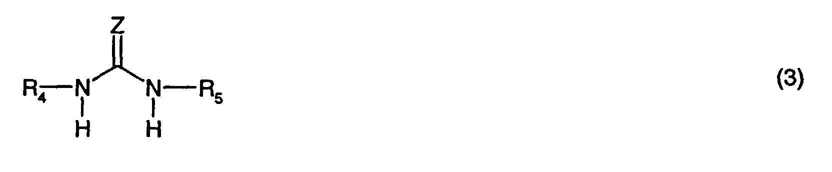 mit Formaldehyd, gegebenenfalls in Gegenwart einer Verbindung der Formel (4) 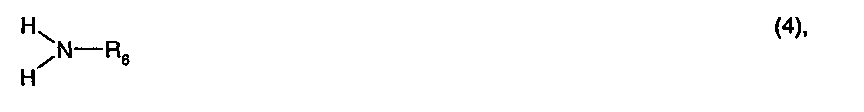 umsetzt und gegebenenfalls eine Sulfonierungsreaktion anschliesst, wobei Z, R₄, R₅ und R₆ die oben angegebenen Bedeutungen und Bevorzugungen haben.

Die N-substituierten Harnstoff- oder Thioharnstoffderivate der Formel (3) sind bekannt oder können in Analogie zu bekannten Verfahren hergestellt werden.

Zur Einführung der Sulfogruppe in den Phenylring der Phenalkylreste R₄, R₅ und/oder R₆ werden die Verbindungen der Formel (2) beispielsweise in Oleum bei etwa 0°C gelöst und dann bei Raumtemperatur mehrere Stunden gerührt.

Die Verwendung der erfindungsgemässen Verbindungen der Formel (2) in dem erfindungsgemässen Verfahren zur Verbesserung der Beständigkeit von Farbstoffen auf natürlichen oder synthetischen Polyamidfasermaterialien gegen Ozoneinwirkung stellt noch einen zusätzlichen Gegenstand der vorliegenden Erfindung dar.

Die nachfolgenden Beispiele dienen zur Erläuterung der Erfindung. Die Temperaturen sind in Celsiusgraden angegeben, Teile sind Gewichtsteile, die Prozentangaben beziehen sich auf Gewichtsprozente, sofern nicht anders vermerkt. Gewichtsteile stehen zu Volumenteilen im Verhältnis von Kilogramm zu Liter.

### Herstellungsbeispiele

Beispiel 1: 12,8 Teile N,N'-Dibenzylthioharnstoff, 15 Teile Formaldehyd und 0,5 Teile konzentrierte Salzsäure werden in 50 Teilen Tetrahydrofuran 24 Stunden bei 50 bis 60°C gerührt. Das auskristallisierte Reaktionsprodukt wird anschliessend abfiltriert und getrocknet. Man erhält 10 Teile der Verbindung der Formel (101)

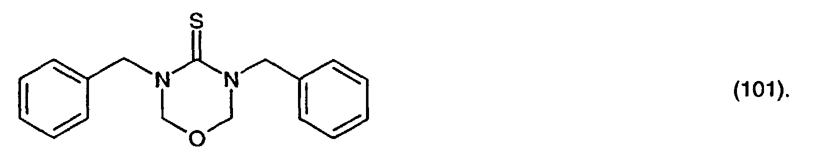

Verwendet man statt 12,8 Teilen N,N'-Dibenzylthioharnstoff die jeweils äquimolare Menge der in Spalte 2 der Tabelle 1 angegebenen Harnstoffderivate, so erhält man die entsprechenden in Spalte 3 der Tabelle 1 angegebenen 1,3,5-Oxadiazin-4-thion- oder -4-onderivate.

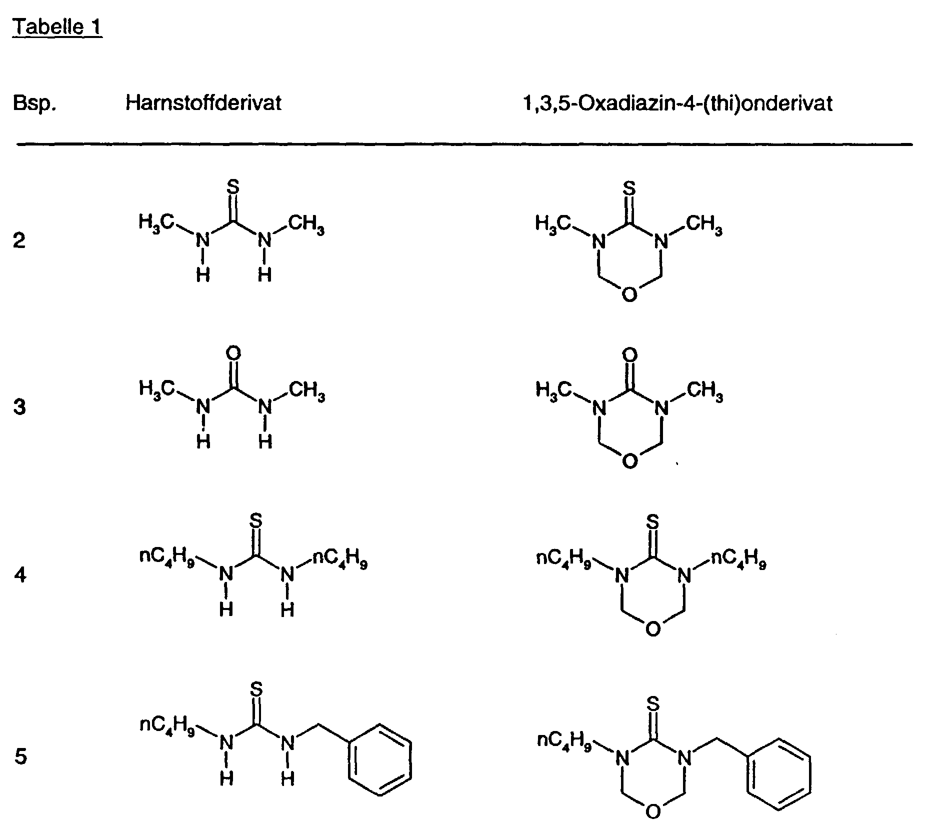

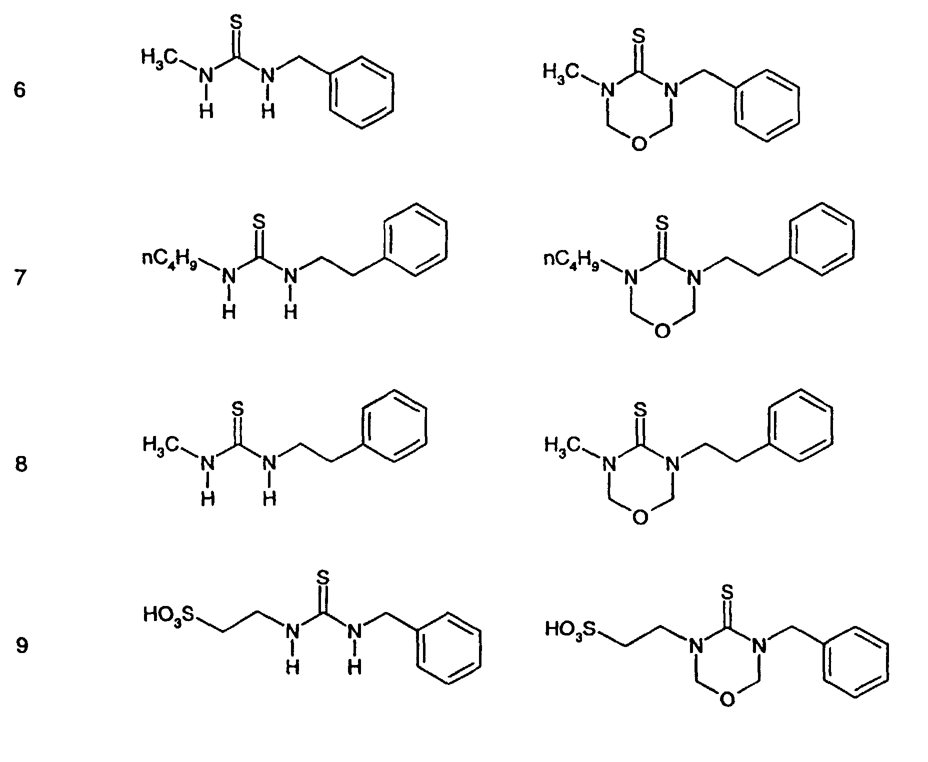

Beispiel 10: 5,2 Teile N,N'-Dimethylthioharnstoff werden in 12,2 Teilen 37%igem, wässrigen Formaldehyd gelöst und mit 5,4 Teilen Benzylamin versetzt. Das Reaktionsgemisch wird dann 24 Stunden bei 60°C gerührt. Das Reaktionsprodukt wird mit Toluol extrahiert. Die organische Phase wird eingedampft und das auskristallisierte Produktwird getrocknet. Man erhält 9 Teile der Verbindung der Formel (102)

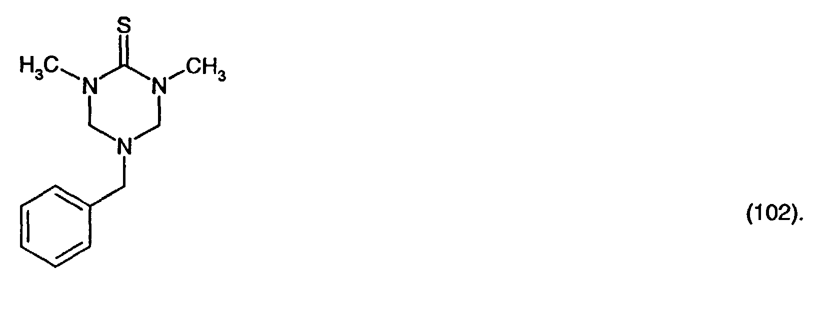

Verwendet man statt 5,2 Teilen N,N'-Dimethylthioharnstoff die jeweils äquimolare Menge der in Spalte 2 der Tabelle 2 angegebenen Harnstoffderivate und anstelle von 5,4 Teilen Benzylamin die jeweils äquimolare Menge der in Spalte 3 der Tabelle 2 angegebenen Amine, so erhält man die entsprechenden in Spalte 4 der Tabelle 2 angegebenen 1,3,5-Triazinthion- oder -onderivate.

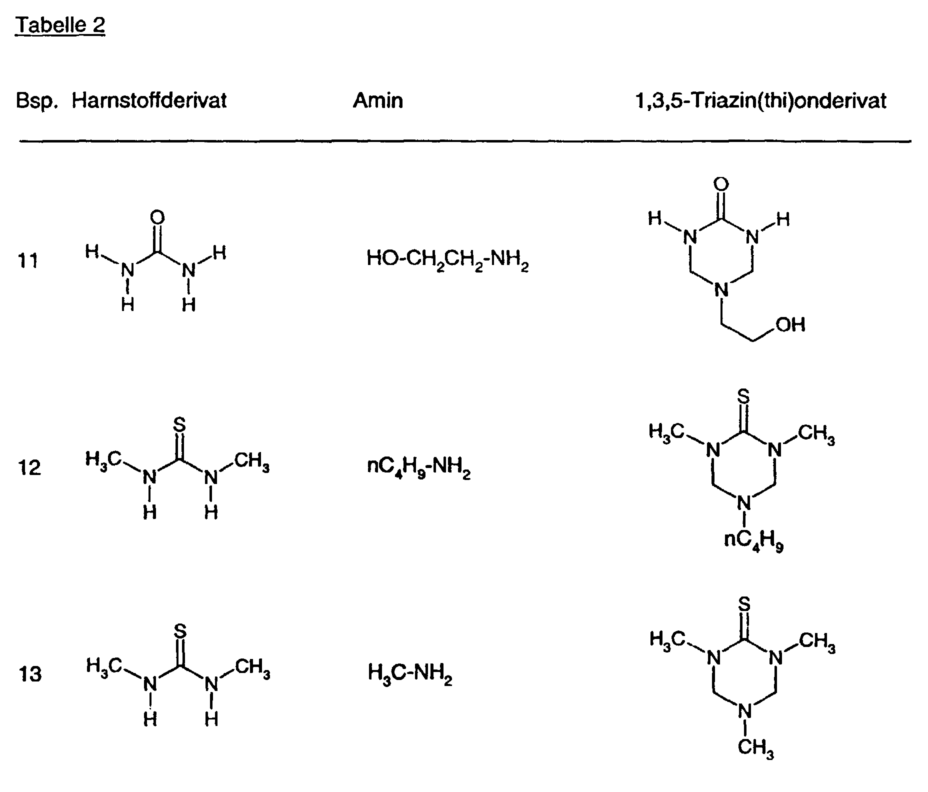

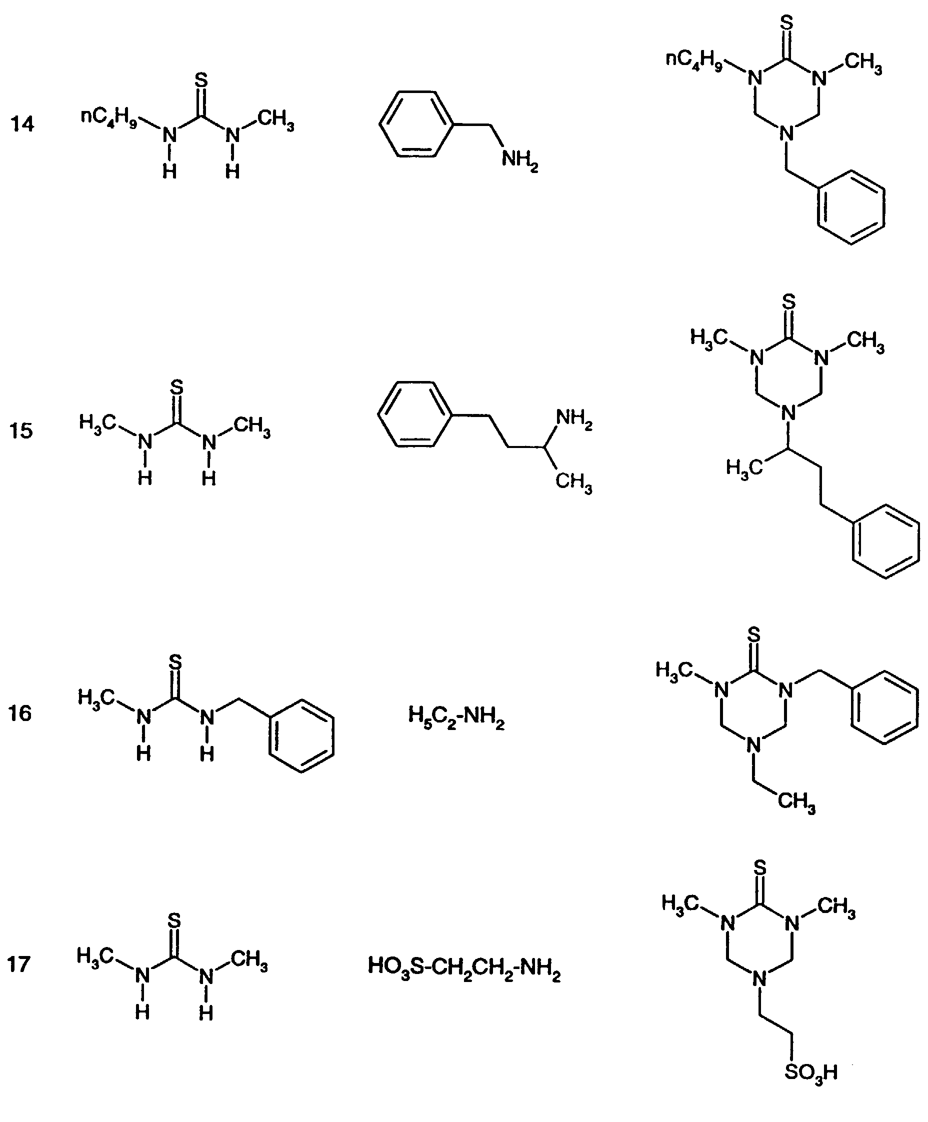

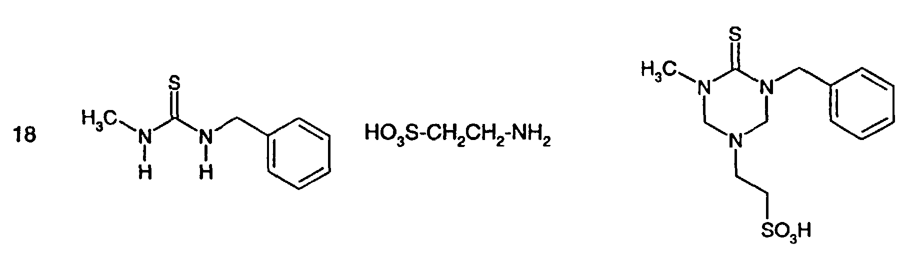

Beispiel 19: 5 Teile der Verbindung gemäss Beispiel 1 werden in 50 Teilen 15%igem Oleum bei 0 bis 5°C gelost. Das Reaktionsgemisch wird dann 5 Stunden bei Raumtemperatur gerührt und anschliessend auf Eis gegossen. Das ausgefallene Reaktionsprodukt wird abfiltriert und getrocknet. Man erhalt 6,4 Teile der Verbindung der Formel (103)

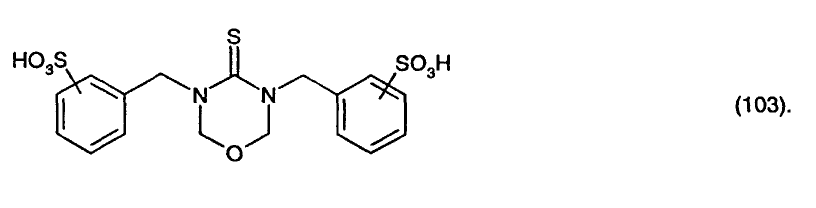

Nach dem gleichen Verfahren können die folgenden in Spalte 3 der Tabelle 3 angegebenen sulfonierten Produkte erhalten werden, wenn man statt der Verbindung gemäss Beispiel 1 jeweils die in Spalte 2 der Tabelle 3 angegebenen Ausgangsprodukte verwendet.

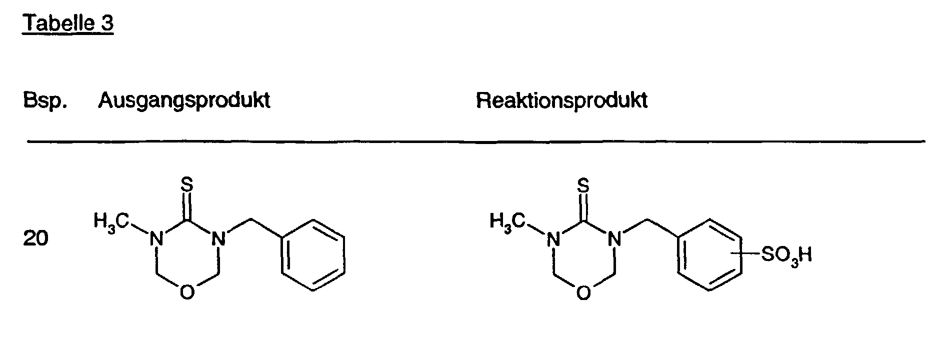

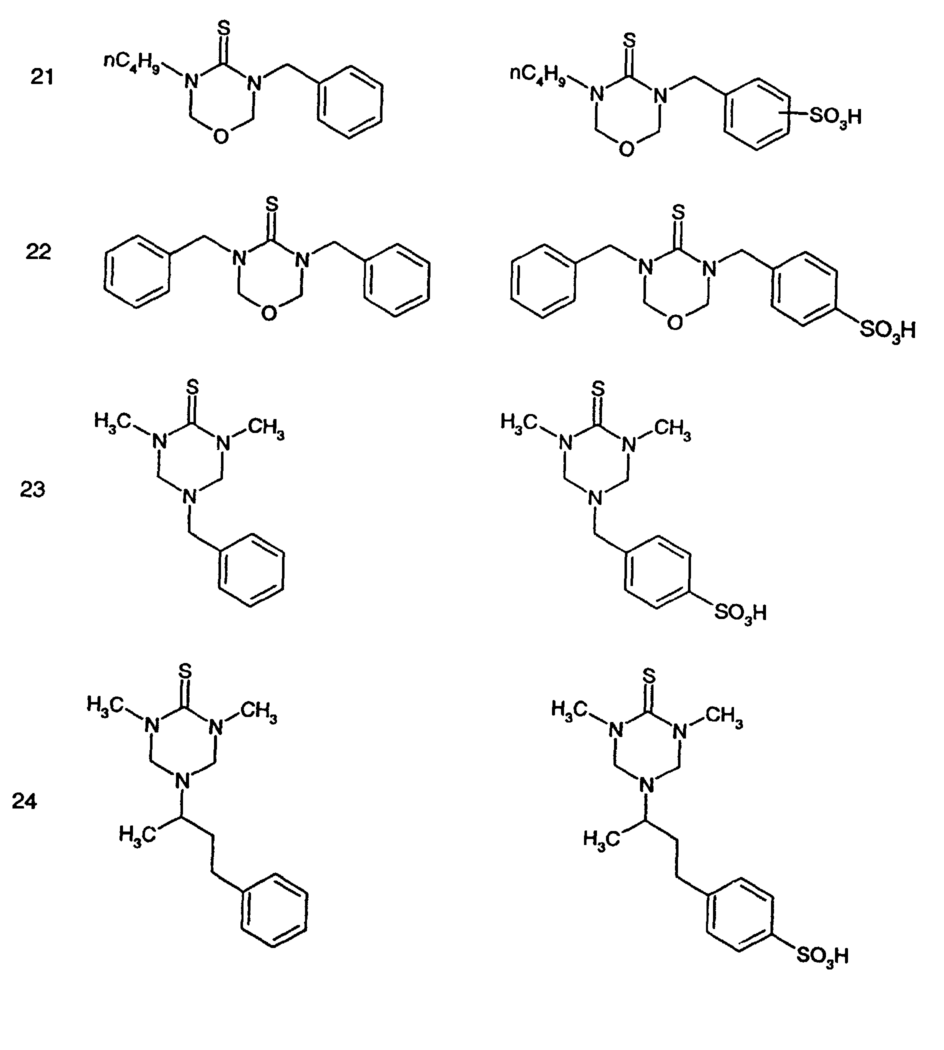

### Anwendungsbeispiele

Beispiel 25: Ein Färbebad enthaltend 600 Teile Wasser, 0,0108 Teile eines Farbstoffs der Formel 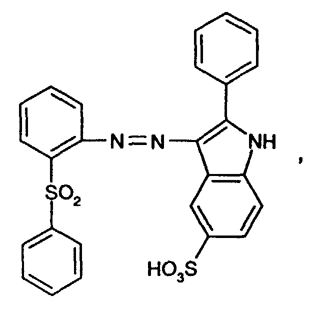 0,0135 Teile eines Farbstoffs der Formel 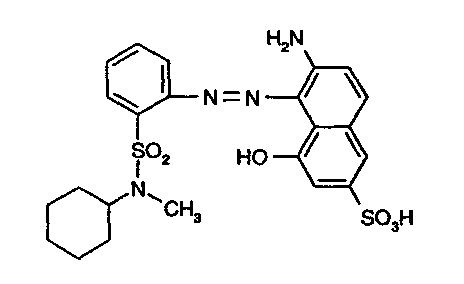 und 0,033 Teile eines Farbstoffs der Formel 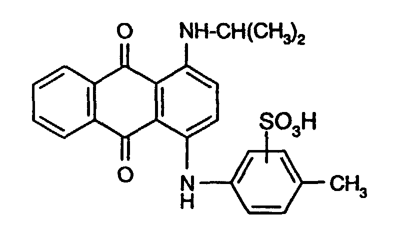 wird mit 0,72 Teilen Natriumdihydrogenphosphat-monohydrat und 0,6 Teilen Dinatriumhydrogenphosphat-dodecahydrat auf einen pH von 6,5 eingestellt. In dieses Färbebad geht man bei 30°C mit 30 Teilen Polyamidteppichgewebe (Polyamid 6) ein. Die Temperatur wird gleichmässig innerhalb von 45 Minuten bis zum Siedepunkt erhöht und man färbt anschliessend noch 30 Minuten bei dieser Temperatur. Der grau gefärbte Teppich wird danach gespült. Das gefärbte Teppichgewebe wird in einem frischen Bad aus 600 Teilen Wasser, 0,6 Teilen der Verbindung gemäss Beispiel 1 (Einsatzmenge 2 Gew.-% bezogen auf das Teppichgewebe), 0,6 Teilen Natriumacetat und 0,7 Teilen Essigsäure bei einem pH von 4,5 und einer Temperatur von 75°C 15 Minuten nachbehandelt. Das Teppichgewebe wird anschliessend gespült und getrocknet. Die Ozonechtheit der erhaltenen Färbung wird nach der Prüfvorschrift ISO 105-G03 durchgeführt. Der Vergleich des nachbehandelten Teppichgewebes mit einem Teppichgewebe, das nicht nachbehandelt wurde, zeigt eine deutliche Erhöhung der Ozonbeständigkeit des nachbehandelten Teppichgewebes.

Verfährt man wie in Beispiel 25 beschrieben, verwendet jedoch anstelle von 2 Gew.-%, bezogen auf das Teppichgewebe, der Verbindung gemäss Beispiel 1 die äquivalente Menge einer der Verbindungen gemäss einem der Beispiele 2 bis 24, so wird ebenfalls ein Polyamidteppichgewebe mit einer grauen, ozonechten Färbung erhalten.

Beispiel 26: Ein Färbebad enthaltend 600 Teile Wasser, 0,0108 Teile eines Farbstoffs der Formel 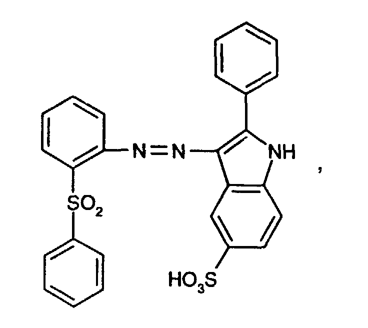 0,0135 Teile eines Farbstoffs der Formel 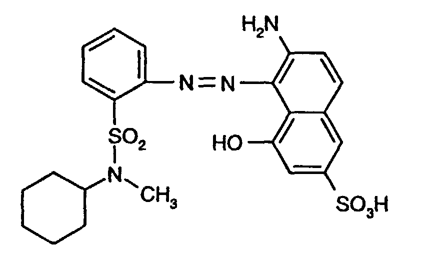 und 0,033 Teile eines Farbstoffs der Formel 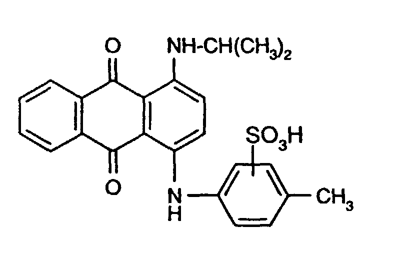 und 0,6 Teile der Verbindung aus Beispiel 1 (Einsatzmenge 2 Gew.-% bezogen auf das Teppichgewebe) wird mit 0,72 Teilen Natriumdihydrogenphosphat-monohydrat und 0,6 Teilen Dinatriumhydrogenphosphat-dodecahydrat auf einen pH von 6,5 eingestellt. In dieses Färbebad geht man bei 30°C mit 30 Teilen Polyamidteppichgewebe (Polyamid 6) ein. Die Temperatur wird gleichmässig innerhalb von 45 Minuten bis zum Siedepunkt erhöht und man färbt anschliessend noch 30 Minuten bei dieser Temperatur. Der grau gefärbte Teppich wird danach kurz kalt gespült und getrocknet. Die Ozonechtheit der erhaltenen Färbung wird nach der Prüfvorschrift ISO 105-G03 durchgeführt. Der Vergleich des gefärbten Teppichgewebes mit einem Teppichgewebe, das nicht in Gegenwart der Verbindung aus Beispiel 1 gefärbt wurde, zeigt eine deutliche Erhöhung der Ozonbeständigkeit.

Verfährt man wie in Beispiel 26 beschrieben, verwendet jedoch anstelle von 2 Gew.-%, bezogen auf das Teppichgewebe, der Verbindung gemäss Beispiel 1 die äquivalente Menge einer der Verbindungen gemäss einem der Beispiele 2 bis 24, so wird ebenfalls ein Polyamidteppichgewebe mit einer grauen, ozonechten Färbung erhalten.

## Patentansprüche

1. Verfahren zur Verbesserung der Beständigkeit von Farbstoffen auf natürlichen oder synthetischen Polyamidfasermaterialien gegen Ozoneinwirkung, dadurch gekennzeichnet, dass man das Fasermaterial vor, während oder nach der Färbung mit einer Flotte, die mindestens eine Verbindung der Formel (1) 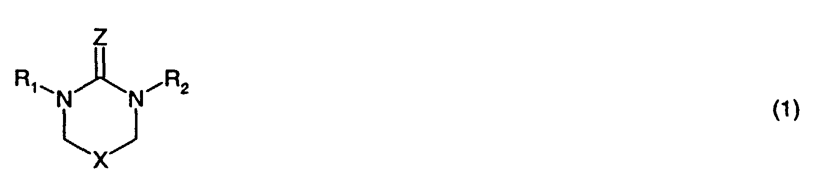 enthält, worin
X Sauerstoff oder einen Rest N-R₃ und
Z Sauerstoff oder Schwefel bedeuten, und
R₁, R₂ und R₃ unabhängig voneinander Wasserstoff, gegebenenfalls substituiertes C₁-C₆-Alkyl, C₅-C₇-Cycloalkyl oder C₇-C₁₂-Phenalkyl sind,
nach dem Auszieh- oder Kontinueverfahren behandelt.

2. Verfahren gemäss Anspruch 1, dadurch gekennzeichnet, dass man das Fasermaterial während oder nach der Färbung behandelt.

3. Verfahren gemäss Anspruch 1 oder 2, dadurch gekennzeichnet, dass die Verbindung der Formel (1) in einer Menge von 0,05 bis 10 Gew.-% und vorzugsweise 0,1 bis 6 Gew.-%, bezogen auf das Gewicht des Polyamidfasermaterials, in der Flotte vorhanden ist.

4. Verfahren gemäss einem der Ansprüche 1 bis 3, dadurch gekennzeichnet, dass man die Behandlung mit der die Verbindung der Formel (1) enthaltenden Flotte bei einem pH-Wert von 2 bis 7 und vorzugsweise von 4 bis 7 durchführt.

5. Verfahren gemäss einem der Ansprüche 1 bis 4, dadurch gekennzeichnet, dass man die Behandlung mit der die Verbindung der Formel (1) enthaltenden Flotte bei einer Temperatur von 50 bis 100°C durchführt.

6. Verfahren gemäss einem der Ansprüche 1 bis 5, dadurch gekennzeichnet, dass es sich bei dem Fasermaterial um synthetisches Polyamidfasermaterial handelt.

7. Verbindungen der Formel (2) 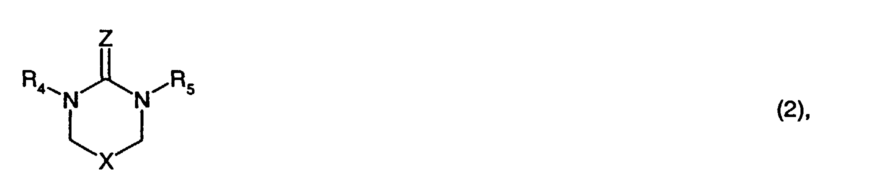 worin
X Sauerstoff oder einen Rest N-R₆ und
Z Sauerstoff oder Schwefel bedeuten, und
R₄, R₅ und R₆ unabhängig voneinander Wasserstoff, gegebenenfalls durch Halogen, Hydroxy, C₁-C₄-Alkoxy, C₂-C₄-Alkanoylamino, Sulfo oder Sulfato substituiertes C₁-C₆-Alkyl, gegebenenfalls durch C₁-C₄-Alkyl substituiertes C₅-C₇-Cycloalkyl oder gegebenenfalls im Phenylteil durch Halogen, C₁-C₄-Alkyl, C₁-C₄-Alkoxy, C₂-C₄-Alkanoylamino oder Sulfo substituiertes C₇-C₁₂-Phenalkyl sind,
mit der Massgabe, dass
mindestens einer der Substituenten R₄ und R₅ gegebenenfalls im Phenylteil durch Halogen, C₁-C₄-Alkyl, C₁-C₄-Alkoxy oder C₂-C₄-Alkanoylamino substituiertes C₇-C₁₂-Phenalkyl bedeutet, oder
mindestens einer der Substituenten R₄, R₅ und R₆ durch Sulfo substituiertes C₁-C₆-Alkyl oder im Phenylteil durch Sulfo substituiertes C₇-C₁₂-Phenalkyl bedeutet.

8. Verbindungen gemäss Anspruch 7, worin
R₄, R₅ und R₆ unabhängig voneinander Wasserstoff, gegebenenfalls durch Hydroxy, C₁-C₄-Alkoxy, Sulfo oder Sulfato substituiertes C₁-C₄-Alkyl oder gegebenenfalls im Phenylteil durch C₁-C₄-Alkyl, C₁-C₄-Alkoxy, C₂-C₄-Alkanoylamino oder Sulfo substituiertes C₇-C₁₀-Phenalkyl sind,
mit der Massgabe, dass
mindestens einer der Substituenten R₄ und R₅ gegebenenfalls im Phenylteil durch C₁-C₄-Alkyl, C₁-C₄-Alkoxy oder C₂-C₄-Alkanoylamino substituiertes C₇-C₁₀-Phenalkyl bedeutet, oder mindestens einer der Substituenten R₄, R₅ und R₆ durch Sulfo substituiertes C₁-C₄-Alkyl oder im Phenylteil durch Sulfo substituiertes C₇-C₁₀-Phenalkyl bedeutet.

9. Verbindungen gemäss Anspruch 7 oder 8, worin
R₄ gegebenenfalls im Phenylteil durch C₁-C₄-Alkyl, C₁-C₄-Alkoxy oder Sulfo substituiertes C₇-C₁₀-Phenalkyl ist, und
R₅ und R₆ unabhängig voneinander Wasserstoff, gegebenenfalls durch C₁-C₄-Alkoxy oder Sulfo substituiertes C₁-C₄-Alkyl oder gegebenenfalls im Phenylteil durch C₁-C₄-Alkyl, C₁-C₄-Alkoxy oder Sulfo substituiertes C₇-C₁₀-Phenalkyl sind.

10. Verbindungen gemäss einem der Ansprüche 7 bis 9, worin
R₄ gegebenenfalls im Phenylteil durch Sulfo substituiertes C₇-C₁₀-Phenalkyl ist, und
R₅ und R₆ unabhängig voneinander Wasserstoff, gegebenenfalls durch Sulfo substituiertes C₁-C₄-Alkyl oder gegebenenfalls im Phenylteil durch Sulfo substituiertes C₇-C₁₀-Phenalkyl sind.

11. Verbindungen gemäss einem der Ansprüche 7 bis 10, worin
mindestens einer der Substituenten R₄, R₅ und R₆ der Verbindung der Formel (2) durch Sulfo substituiert ist.

12. Verfahren zur Herstellung der Verbindungen der Formel (2) gemäss Anspruch 7, dadurch gekennzeichnet, dass man eine Verbindung der Formel (3) 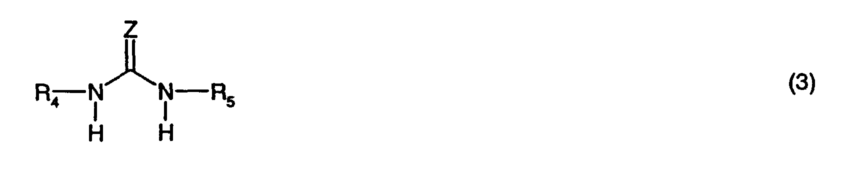 mit Formaldehyd, gegebenenfalls in Gegenwart einer Verbindung der Formel (4) 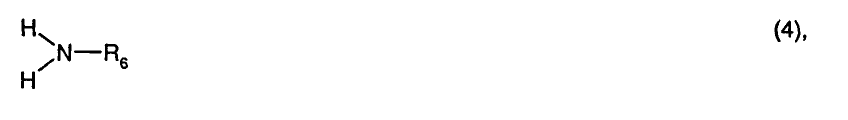 umsetzt und gegebenenfalls eine Sulfonierungsreaktion anschliesst, wobei Z, R₄, R₅ und R₆ die in Anspruch 7 angegebenen Bedeutungen haben.

13. Verwendung der Verbindungen der Formel (2) gemäss einem der Ansprüche 7 bis 11 in einem Verfahren zur Verbesserung der Beständigkeit von Farbstoffen auf natürlichen oder synthetischen Polyamidfasermaterialien gegen Ozoneinwirkung gemäss Anspruch 1.
